# EUROPEAN PATENT SPECIFICATION

(11) **EP 1 221 970 B1**
(45) Date of publication and mention of the grant of the patent: **19.02.2014**
(21) Application number: 00992122.2
(22) Date of filing: 18.10.2000
(51) Int. Cl.: A61K 39/145, A61K 39/39

(54) **COMPOSITIONS AND METHODS FOR STIMULATING AN IMMUNE RESPONSE AGAINST INFECTIOUS AGENTS**
ZUSAMMENSETZUNGEN UND VERFAHREN ZUR STIMULIERUNG EINER IMMUNANTWORT GEGEN INFEKTIÖSE KRANKHEITSERREGER
COMPOSITIONS ET METHODES DESTINES A STIMULER UNE REPONSE IMMUNE CONTRE DES AGENTS INFECTIEUX

(30) Priority: 18.10.1999 US 160028 P
(43) Date of publication of application: 17.07.2002
(73) Proprietor: Novartis Vaccines and Diagnostics, Inc., Emeryville, CA 94608 (US)
(72) Inventor: BARACKMAN, John, D., Dublin, CA 94568 (US); OTT, Gary, Oakland, CA 94605 (US); PINE, Samuel, Oakland, CA 94611 (US); O'HAGAN, Derek, Berkeley, CA 94705 (US)
(74) Representative: Hallybone, Huw George
(86) International application number: PCT/US2000/041241
(87) International publication number: WO 2001/035993

(56) References cited:
- WO-A-99/26654
- WO-A1-98/42375
- BARACKMAN J.D. ET AL.: "Intranasal immunization of mice with influenza vaccine in combination with the adjuvant LT-R72 induces potent mucosal and serum immunity which is stronger than that with traditional intramuscular immunization." INF. IMMUN., vol. 37, no. 8, August 1999 (1999-08), pages 4276-4279, XP002176322
- DOUCE G. ET AL.: "Genetically detoxified mutants of heat-labile toxin from E. coli are able to act as oral adjuvants", INFECTION AND IMMUNITY, vol. 67, no. 9, 1999, pages 4400-4406,
- KATZ J. ET AL.: "Heat-lable enterotoxin from Escherichia coli as an adjuvant for oral influenza vaccination", INTERNATIONAL CONGRESS SERIES, EXCERPTA MEDICA, vol. 1123, 1996, pages 292-297, AMSTERDAM
- O'HAGAN D.T.: "Recent advances in immunological adjuvants: the development of particulate antigen delivery systems", EXPERT OPIN INVESTIG DRUGS, vol. 7, no. 3, March 1998 (1998-03), pages 349-359,
- DEL GIUDICE G. ET AL.,: 'Mucosal delivery of vaccines' METHODS vol. 19, pages 148 - 155
- BARCHFELD G.L. ET AL.: "The adjuvants MF59 and LT-K63 enhance the mucosal and systemic immunogenicity of subunit influenza vaccine administered intranasally in mice", VACCINE, vol. 17, 26 February 1999 (1999-02-26), pages 695-704,
- SONG J.-H.: "Sublingual vaccination with influenza virus protects mice against lethal viral infection.", PROC NATL ACAD SCI USA, vol. 105, no. 5, 2008, pages 1644-1649,
- BAGLEY K.C. ET AL.: "An enzymatically active A domain is required for Cholera-like enterotoxins to induce a long-lived blockade on the induction of oral tolerance: New methods for screening mucosal adjuvants.", INFECT. IMMUN., vol. 72, no. 12, 2003, pages 6850-6856,

## Description

### Field of the Invention

The invention relates to compositions and uses for stimulating an immune response against influenza. Specifically, the invention relates to oral compositions containing hemagglutinin antigen from an influenza virus and at least one heat-labile, mutant *Escherichia coli* enterotoxin as defined in the claims.

### Background of the Invention

Infectious diseases are responsible for significant morbidity and mortality throughout the world. Treatment of infectious disease often is initiated after the patient has been infected and has already suffered from the effects of infection. There has long been a need to develop strategies to prevent infection before the deleterious effects of infections occur.

The majority of infectious disease is acquired via mucosal surfaces. Secretory immunoglobulins A (IgA) may function as a first line of defense against such infections, preventing attachment and transmission through the mucosa, and may inhibit viral replication within infected epithelial cells.

One infectious disease of particular importance is influenza. Influenza is a serious human disease exhibiting high mortality in vulnerable populations such as the very young, the very old, and immune compromised individuals, as well as significant morbidity in the general population (Glezen P.W. (1982) Epidemiol. Rev. 4:25-44). The social and economic costs associated with yearly influenza outbreaks are high (Clements M.L., and I. Stephens. "New and improved vaccines against influenza." in NEW GENERATION VACCINES, 2nd edition (Levine M.M., et al.. Eds.). Marcel Dekker. Inc., New York, 1997. pp. 545-570). Formalin-inactivated whole virus and split-virus intramuscular (i.m.) vaccines are commercially available to control the spread and severity of influenza (Ghendon Y. (1989) Adv. Exp. Med. Biol. 257:37-45; Riddiough M.A. et al. (1983) JAMA 249:3189-95). These prophylactic vaccines, although important agents in controlling influenza, suffer from a number of shortcomings that limit their efficacy and acceptability. Current commercial influenza vaccines are shown to induce serum antibody responses in healthy adult humans that are protective against viral challenge, but, this protective immunity tends to be variable in potency and is relatively short lived, particularly in the elderly and infant populations (Clements M.L. and I. Stephens (1997) pp. 545-70; Ghendon Y. (1989) Adv. Exp. Med. Biol. 257:37-45; Riddiough M.A. et al. (1983) JAMA 249:3189-3195; Hoskins T.W. (1979) Lancet i:33-35; Patriarca P.A. et al. (1985) JAMA 253:1136-1139). Moreover, inactivated whole virus and split-virus vaccines are known to activate CD8⁻ cytotoxic T-lymphocyte (CTL) responses only sporadically, have poor cross-reactivity to antigenic variants, and produce poor secretory IgA responses (Glezen P.W. (1982) Epidemiol. Rev. 4:25-44; Clements M.L., and I. Stephens (1997) pp. 545-570; Bender B.S. et al. (1991) Immunol. 72:514-519; Hoskins, T.W. (1979) Lancet i:33-5; Patriarca P.A. et al. (1985) JAMA 253:1136-39; Powers D.C. (1993) J. Am. Geriatr. Soc. 41:1-5). Furthermore, injection site reactogenicity and weak immune responses can be a problem in very young children (Groothuis J.R. et al. (1994) Vaccine 12:139-41; Groothuis J.R. et al. (1991) Pediatrics 87:823-828). Significant efforts are currently being pursued to improve the vaccines efficacy and tolerability primarily through development of mucosally active influenza vaccines (Clements M.L., and I. Stephens (1997) pp. 545-570; Barackman J.D. et al. (1999) Infect. Immun. 67:4276-4279; De Haan A. et al. (1995) Vaccine 13:155-162; Oh Y. et al. (1998) Vaccine 10:506-511; Santiago N. et al. "Vehicles for oral immunization" in VACCINE DESIGN: THE SUBUNIT AND ADJUVANT APPROACH. Powell F.M. and M.J. Newman (Eds.). Plenum Press. New York. 1995. pp. 413-38).

Mucosal immunization strategies have been extensively investigated as a means to improve the efficacy and duration of influenza vaccination by providing a broader immune response than that afforded by i.m. immunization (Oh Y. et al. (1998) Vaccine 10:506-511; Gallichan W.S. and K.L. Rosenthal (1996) J. Exp. Med. 184:1879-1890; Ogra P.L. "Mucosal immunoprophylaxis: an introductory overview" in MUCOSAL VACCINES, Kiyono H. et al. (Eds.). Academic Press, New York, 1996. pp. 3-14; Novak M. et al. (1995) Adv. Exp. Med. Biol. 371B: 587-1590; Staats H.F. and J.R. McGhee "Application of basic principles of mucosal immunity to vaccine development" In MUCOSAL VACCINES, Kiyono H. et al. (Eds.). Academic Press, New York, 1996. pp. 17-39). The most commonly employed, and thus far most successful, mucosal immunization strategy for influenza vaccination is via the intranasal route (Barackman J.D. et al. (1999) Infect. Immun. 67:4276-4279; Nichol K.L. et al. (1999) JAMA 282:137-144; Rudin A. et al. (1998) Infect. Immun. 66:3390-3396; Takase H. et al. (1996) Vaccine 14:1651-1656).

Intranasal immunization with live, cold-adapted influenza virus vaccines, and co-administration of influenza antigens with LT (heat-labile enterotoxin) and CTB (the non-toxic B subunit of cholera toxin) appear to be viable approaches to development of improved influenza vaccines (Dickinson B.L. and J.D. Clements "Use of Escherichia coli heat-labile enterotoxin as an oral adjuvant" in MUCOSAL VACCINES. Kiyono H. et al. (Eds.). Academic Press, New York, 1996. pp. 73-87; Nichol K.L. et al. (1999) JAMA 282:137-144). These approaches have been shown in humans to increase local (salivary and upper respiratory) antigen-specific IgA levels, as well as increased cellular immune responses compared to traditional i.m. immunization with the commercial influenza vaccines. The live, cold-adapted influenza virus vaccines have been shown, however, to have limited efficacy in elderly patients, and have been shown to be poor CTL stimulators in infants (Mbawuike I.N. et al. (1996) J. Med. Virol. 50:105-111; Treanor J. et al. (1994) J. Infect. Dis. 169:402-407). Toxicity has been the primary limiting factor for use of enterotoxins as mucosal adjuvants in humans. Intranasal administration of influenza antigens with LT-K63 is known from Barchfeld et al. (1999) Vaccine 17: 695-704. A combination of influenza hemagglutinin and LT mutants is known from WO98/42375 for parenteral administration.

Oral immunization has long been a desirable target for vaccination. Like intranasal immunization, oral immunization has been shown to induce strong secretory IgA responses, improve protective cellular immune responses, and result in significant serum antibody responses as well (Takase H. et al. (1996) Vaccine 14:1651-1656: Benedetti R. et al. (1998) Res. Immunol. 149:107-118; Gallichan W.S. and K.L. Rosenthal (1996) J. Exp. Med. 184:1879-1890; Novak M. et al. (1995) Adv. Exp. Med. Biol. 371B:1587-1590; Katz J.M. et al. (1997) J. Infect. Dis. 175:352-363). The secretory IgA responses for oral immunization have been shown in some animal models to be strongest in the urogenital and rectal tracts, and, when compared to intranasal immunization, somewhat muted upper respiratory, nasopharyngeal, and saliva responses (Rudin A. et al. (1998) Infect. Immun. 66:3390-3396). These relatively weak upper respiratory IgA responses, if found to be the case in the human system, would seem to be a problem with respect to achieving effective protection against viral challenge against viruses whose primary mode of entry is via the upper respiratory tract (such as influenza). Other studies, however, have shown there is sufficient local secretory IgA responses, and more importantly, evidence of antigen primed B and T cell migration to the upper respiratory sites to induce potent protective immunity (Takase H. et al. (1996) Vaccine 14:1651-1656; Katz J.M. et al. (1997) J. Infect. Dis. 175:352-363). Furthermore, oral immunization has been shown to promote memory B-cell maintenance in the bone marrow, a factor that may be important in the development of the persistence of immunity against viral challenge (Benedetti R. et al. (1998) Res. Immunol. 149:107-118), However, to obtain strong immune responses from many antigens, a potent mucosal adjuvant, usually an enterotoxin, must be co-administered (De Aizpurua H.J. et al. (1998) J. Exp. Med. 167:440-451). Del Giudice et al. (1999) Methods, 19: 148-55 teaches intragastric administration of antigens with LT-K63 but does not mention influenza.

Studies have shown that immune responses to orally immunized antigens were significantly stronger if the antigen by itself had mucosal binding properties, orcould be made to have mucosal binding properties by chemically coupling to agents with mucoadhesive, lectin, or receptor-binding properties (Harokopakis E. et al. (1998) Infect. Immun. 66:4299-4304; Neutra M.R. and J. Kraekenbuhl "Antigen uptake by M cells for effective mucosal vaccines" in MUCOSAL VACCINES Kiyono H. et al. Eds., Ogra PL. McGhee JR, eds. Mucosal Vaccines. New York: Academic Press 1996:41-55, De Aizpurua H.J. and G.J. Russell-Jones (1988) J. Exp. Med. 167:440-451; Czerkinsky C. et al. (1989) Infec. Immun. 57:1072-1077). CTB has been used for these purposes with some success (De Aizpurua H.J. and G.J. Russell-Jones (1988) J. Exp. Med. 167:440-451; Czerkinsky C. et al. (1989) Infec. Immun. 57:1072-1077). Influenzahemagglutinin (HA) are membrane glycoproteins from influenza viruses that agglutinate erythrocytes, and mediate viral attachment and envelope fusion. Influenza HA binds neuraminic acid rich glycoproteins, while LT-R72 and LT-K63 binds GM₁-ganglioside, as well as galactose containing glycoproteins and lipopolysaccharides, all of which ligands are found ubiquitously in the gut (Kuziemko G.M. et al. (1996) Biochem. 35:6375-6384; Pritchett T.J. et al. (1987) Virology, 160:502-506: Spangler B.D. (1992) Microbiol. Rev. 56:622-647).

Although numerous obstacles make oral immunization using subunit antigens a significant challenge, it is considered by many to be a highly desirable form of vaccination (Barackman J.D. et al. (1998) STP Pharma. Sci. 8:41-46; Challacombe S.J. et al. (1992) Immunol. 76:164-168; Dickinson B.L. and J.D. Clements. "Use of Escherichia coli heat-labile enterotoxin as an oral adjuvant" in MUCOSAL VACCINES, Kiyono H. et al. (Eds.) Academic Press, New York, 1996. pp. 73-87). The potential of oral vaccination to generate strong cellular immunity, better cross-protection, memory, and secretory IgA responses have been postulated (Takase H. et al. (1996) Vaccine 14:1651-1656; Benedetti R. et al. (1998) Res. Immunol. 149:107-118; Gallichan W.S. and K.L. Rosenthal (1996) J. Exp. Med. 184:1879-1890: Meitin C.A. et al (1994) Proc. Natl. Acad. Sci. USA 91:11187-11191: Ogra P.L. "Mucosal immunoprophylaxis; an introductory overview" in MUCOSAL VACCINES Kiyono H. et al. (Eds.) Academic Press, New York. 1996. pp. 3-14), although the added benefit of patient comfort cannot be over-emphasized (Rahman S. et al. (1993) Am. J. Trop. Med. Hyg. 48:823-826).

Administration of an influenza vaccine in the form of a chewable pill or palatable sweet liquid formulation is considered by many to be the preferred form of administration in children, and is safer than injectables for the clinician. Many approaches have been investigated to develop viable orally active influenza vaccines including formulation of influenza antigens into microparticles, coupling antigens to carrier proteins that target cellular uptake into Payer's Patches, expression of influenza antigens in bacterial and viral vectors, and co-administration with mucosally active adjuvants (Barackman J.D. et al. (1998) STP Pharma. Sci. 8:41-46: Meitin C.A. et al. (1994) Proc. Natl. Acad. Sci. USA 91:11187-11191; Harokopakis E. et al. (1998) Infect. Immun. 66:4299-4304; Neutra M.R. and J. Kraekenbuhl. "Antigen uptake by M cells for effective mucosal vaccines" in MUCOSAL VACCINES Kiyono H. et al., Eds.) Academic Press. New York, 1996. pp. 41-55). Of these approaches, the mucosal adjuvants, primarily *Escherichia coli* heat-labile enterotoxin (LT) and cholera toxin (CT) are the most commonly employed (Dickinson B.L. and J.D. Clements "Use of Escherichia coli heat-labile enterotoxins as an oral adjuvant" in MUCOSAL VACCINES Kiyono H. et al., (Eds.) Academic Press, New York, 1996. pp. 73-87; Elson C.O. "Cholera toxin as a mucosal adjuvant" in MUCOSAL VACCINES Kiyono H. et al., (Eds.) Academic Press. New York. 1996. pp. 59-72). Although potent mucosal adjuvants. LT and CT are toxic in humans at doses useful for adjuvanticity, and, therefore, are not useful for developing oral influenza vaccines.

The non-toxic B-subunit ofCT (CTB) has been investigated as an alternative to whole CT. however, studies have indicated small amounts of the whole CT are required for sufficient adjuvant potency, inhibiting the potential of CTB in humans (Tamura S. et al. (1991) Eur. J. Immunol. 21:1337-1344; Tamura S. et al. (1992) J. Immunol. 149:981-988; Tamura S. et al. (1994) Vaccine 12:1083-1089). Because of these studies, the ADP-ribosyltransferase activity of LT and CT have been implicated as a necessary component for adjuvanticity (Lyche N. et al. (1992) Eur. J. Immunol. 22:2277-81).

There is a need in the art to develop compositions which safely elicit an immune reaction when administered orally.

### Summary of the Invention

The present invention provides for compositions that elicit an immune response in a mammal wherein the compositions contain influenza virus hemagglutinin and a LT-K63 or LT-R72 mutant of E. coli heat labile toxin, in a pharmaceutically acceptable carrier for oral use, as defined in the claims.

An oral influenza immunogenic composition for mammals is provided in which the immunogenic composition contains an effective amount of an influenza hemagglutinin and a LT-K63 or LT-R72 heat-labile *Escherichia coli* enterotoxin.

### Brief Description of the Figures

**Fig. 1** shows a comparison of enterotoxin dose on antigen-specific serum antibody responses after intragastric (i.g.) administration.
**Fig. 2** shows a comparison of enterotoxin dose on antigen-specitic saliva wash (SW) IgA responses after i.g. administration.
**Fig. 3** shows a comparison of HA dose on antigen-specific serum antibody responses after i.g. administration.
**Fig. 4** shows a comparison of HA dose on antigen-specific saliva wash (SW) IgA responses after i.g. administration.
**Fig. 5** shows a comparison of the effects of i.m. and i.g. administration of A/Johannesburg/97 HA on antigen-specific serum antibody responses.
**Fig. 6** shows a comparison of the effects of i.m. and i.g. administration of A/Johannesburg/97 HA on serum HI titers.
**Fig. 7** shows a comparison of the effects of i.m. and i.g. administration of A/Johannesburg/97 HA on antigen-specific nasal wash (NW) IgA antibody responses.

### Detailed Description of the Invention

It has been discovered that antigens that do not have any mucoadhesive or gut-associated binding properties have minimal immunogenicity when delivered orally in mice, other than at very high dose levels, either in the absence of LT's or as mixtures of soluble antigen with soluble LT. Modest immune responses may be shown when influenza antigens are delivered orally at reasonable dose levels, but these antigens result in substantial and broad immune responses when adjuvanted with wild-type LT or CT (Katz J.M. et al. (1997) J. Infect. Dis. 175:352-363).

We have studied the mutant LT toxins LT-K63 and LT-R72 (Barackman J.D. et al. (1999) Infect. Immun. 67:4276-4279). These toxins demonstrate similar adjuvanticity to that of wtLT when delivered intranasally in combination with influenza antigens, yet demonstrate substantially reduced to zero *in vitro* and *in vivo* toxicity, improving the odds of developing broadly applicable, effective intranasal influenza vaccine (Barackman J.D. et al. (1999) Infect. Immun. 67:4276-4279; Giuliani M.M. et al. (1998) J. Exp. Med. 187:1123-1132). Furthermore, LT-R72 exhibits extremely low levels of ADP-ribosyltransferase activity yet maintains potent mucosal adjuvant activity, while ADP-ribosyltransferase activity is undetectable in LT-K63 despite potent adj uvanticity (Giuliani M.M. et al. (1998) J. Exp. Med. 187:1123-1132). The data demonstrate that ADP-ribosyltransferase activity may not be linked to the adjuvant activity, rendering these adjuvants suitable, non-toxic mucosal adjuvants for oral administration in humans (Freytag L.C. and J.D. Clements (1999) Curr. Top. Microbiol. Immunol. 236:215-236).

The practice of the present invention will employ, unless otherwise indicated, conventional methods of virology, immunology, microbiology, molecular biology and recombinant DNA techniques within the skill of the art. Such techniques are explained fully in the literature. See. e.g., Sambrook, et al., Molecular Cloning: A Laboratory Manual (2nd Edition. 1989); DNA CLONING: A PRACTICAL APPROACH. Vols. I & II (D. Glover, ed.); METHODS IN ENZYMOLOGY (S. Colowick and N. Kaplan eds., Academic Press. Inc.); HANDBOOK OF EXPERIMENTAL IMMUNOLOGY, Vols. I-IV (D.M. Weir and C.C. Blackwell eds., Blackwell Scientific Publications); and FUNDAMENTAL VIROLOGY, 2nd Edition, Vols. I & II (B.N. Fields and D.M. Knipe, eds.).

As used herein, the terms "a," "an." and "the" refer to the singular and the plural.

As used herein "mucoadhesive" refers to an immunogenic compound that is found associated with an infectious agent of the muscosa and/or alimentary canal in which the antigen has gut-associated or mucosal binding properties.

As used herein "mucosa" refers to the lubricated inner lining of the mouth, nasal passages, vagina and urethra, and "alimentary canal" refers to the digestive tract extending from the mouth to the anus.

An effective amount of the composition of the invention is administered to a mammal in order to prevent or ameliorate infection with an infectious agent. As used herein, the phrase "effective amount" in reference to treating an individual having a disease or condition, means a quantity sufficient to effectuate treatment and ameliorate and/or eliminate the disease or condition, or to prevent an infection with an infectious agent, without untoward effects such as toxicity, irritation or an allergic response. Although individual needs may vary and some variation of dosage requirements will be necessary for different types of mammals to achieve optimal ranges of effective amounts of formulations, such routine experimentation is in the purview of the skilled artisan. Human doses can readily be extrapolated from animal studies as taught by Katocs et al., Chapter 27 of REMINGTON'S PHARMACEUTICAL SCIENCES, 18th Edition, Gennaro (Ed.) Mack Publishing Co., Easton, PA, 1990. Generally, the dosage required to provide an effective amount of a formulation, which can be adjusted by one skilled in the art, will vary depending on several factors, including the age, health, physical condition, weight, type and extent of the disease or disorder of the recipient, frequency of treatment, the nature of concurrent therapy, if required, and the nature and scope of the desired effect(s) (Nies et al., Chapter 3, GOODMAN & GILMAN'S THE PHARMACOLOGICAL BASIS OF THERAPEUTICS, 9th Ed.. Hardman et al., Eds., McGraw-Hill, New York, NY. 1996). A dosage in the range of about 5 to about 100 µg is contemplated.

It is also contemplated that more than one administration of the compositions may be required. The time between administrations depends upon the number of administrations to be given. For example, if two administrations are given, the first can occur at zero months and the second can occur at one, two, or six months: if four administrations are given, they can occur at 0, 1. 2, and 6 months, respectively. Alternatively, the administrations can occur at monthly intervals. The time between multiple administrations can be readily determined by one skilled in the art.

As used herein, the term "administering" includes, but is not limited to, transdermal, parenteral, subcutaneous, intra-muscular, oral, and topical delivery. In the method of the present invention, at least one administration is oral, and the preferred route of administration is oral. The compositions of the present invention are preferably formulated for oral administration.

The intended purpose of the methods of the disclosed invention is the amelioration of infection with influenza viruses. Amelioration can be determined by, for example, a decrease in signs and symptoms of infection associated with influenza. Effective immunization with against influenza may be monitored by serum testing wherein antigen-specific antibodies are elicited. In such tests, antibodies may be detected in the blood, saliva and nasal secretions of the subject using routine tests. Preferably, the treatment according to the invention will result in the appearance of antigen-specific anti-influenza antibodies, with the concurrent decrease in/disappearance of the influenza virus. In some embodiments, treatment with the immunogenic composition may prevent infection with influenza virus. In some embodiments of the invention, the assays may detect the presence of antigen-specific IgA antibodies.

Serum assays described herein may be used to assist in determining effective dosages for the subjects. Sufficient stimulation of immune responses may be determined through immunologic assay, such as Enzyme-Linked Immunosorbent Assays (ELISA) or any other assays to detect antigen-specific antibodies in bodily fluids such as serum, saliva and nasal secretions. Correlating concentration of antigen in the compositions of the invention with antibody titers provides an index of efficacy in the ability of the composition to elicit an effective immune response.

As used herein, the phrase "immunologically effective amount" in reference to immunogenic compositions, means a quantity sufficient to induce a therapeutic or prophylactic immune response.

As used herein, the phrase "prophylactic immune response" in reference to treating an individual against infection by an infectious agent, means an immune response that is prophylactic and inhibits the infectious agent upon challenge.

As used herein "inhibits" in reference to a prophylactic immune response, means to reduce or eliminate infection with the infectious agent such that the effects of infection are minimized or eliminated.

As used herein, the phrase "therapeutic immune response" in reference to treating an individual infected with an infectious agent, means an immune response that ameliorates and/or eliminates the infectious agent.

As used herein, the phrase "therapeutically effective amount" in reference to the amount of an immunogenic composition administered to an individual, means a quantity sufficient to induce a therapeutic immune response in the individual.

As used herein, the phrase "prophylactically effective amount" in reference to the amount of an immunogenic administered to an individual, means a quantity sufficient to induce a prophylactic immune response in the individual.

As used herein. "individual" refers to human and non-human animals that can be treated with the immunogenic compositions of the invention.

"Infectious agents of the mucosa or alimentary canal" include, but are not limited to viruses, bacteria, protozoans, fungi, and helminths. The invention relates to influenza viruses.

The mucoadhesive influenza hemagglutinin antigen of the present invention may be prepared by any means known in the art. Whole pathogens may be inactivated, killed, sonicated, and/or solublized, for example, and the antigens extracted. Antigen preparations for use in the invention may be further purified using conventional methods. Alternatively, antigens may be produced by recombinant DNA technology wherein a nucleic acid sequence encoding a selected antigen is inserted into an expression vector which is subsequently introduced into a host cell. Expression of the recombinant protein is effected and the selected antigen is purified from the host cells by conventional methods. Such methods may include affinity purification, chromatography, and electrophoresis, for example.

The form of the oral compositions of the invention may be capsules, tablets, liquids, syrups, suspensions, elixirs or any other formulation of oral administration known in the art. In addition, the oral compositions of the invention may be combined with other excipients known and used in the art. The compositions may be in the form of ingestible tablets, buccal tablets, troches, capsules, elixirs, suspensions, syrups, wafers, and the like, LT-K63 or LT-R72 may be used at a total dose of about 10 µg to 10 mg. The adjuvant may form a portion of a total oral formulation of about 0.01 to 1% of the total formulation. The dose of the excipients, including the mucoadhesive, may be 100 to 1000 times more than the adjuvant dose. The amount of mucoadhesive antigen in a therapeutically useful composition is that which is sufficient to elicit a therapeutically effective immune response or an infection inhibiting immune response.

The tablets, troches, pills, capsules and the like may also contain the following ingredients: a binder such as polyvinylpyrrolidone, gum tragacanth, acacia, sucrose, corn starch, gelatin and the like; an excipient such as calcium phosphate, sodium citrate, calcium carbonate and the like; a disintegrating agent such as corn starch, potato starch, tapioca starch, certain complex silicates, alginic acid, and the like; a lubricant such as sodium lauryl sulfate, talc, magnesium stearate and the like; a sweetening agent such as sucrose, lactose, saccharin and the like; or a flavoring agent such as peppermint, oil of wintergreen, cherry flavoring, or any other flavoring known and used in the art. Solid compositions of a similar type are also employed as fillers in soft and hard-filled gelatin capsules. When the dosage unit form is contained in a capsule, the composition may be present in a liquid carrier.

Various other materials may be present as coatings orto otherwise modify the physical form of the dosage unit. For instance, tablets, pills, or capsules may be coated with shellac, sugar or both.

A syrup or elixir containing the composition of the invention, may also contain a sweetening agent, preservatives (*e.g.*, methyl and propylparabens), a dye, a flavouring, emulsifying agents and/or suspending agents, and diluents (*e.g.*, water, ethanol, propylene glycol, glycerin and various combinations thereof known and used in the art).

Dosage units are preferably pure and produced under good manufacturing practice (GMP) conditions.

In a preferred embodiment of the invention, a immunogenic amount of at least one influenza hemagglutinin antigen is combined with an effective amount of at least one heatlabile, mutant *Escherichia coli* enterotoxin to form an immunogenic composition against influenza virus. The composition is for oral administration to mammals, particularly humans, to elicit an immune response against influenza. More specifically, an IgA-specific immune response is elicited against influenza, and anti-influenza IgA antibodies are found in the saliva and nasal secretions of the mammal that receives the immunogenic composition. The enterotoxin used in the composition is LT-K63, LT-R72 or mixtures thereof. Administration of the immunogenic composition is via the oral route.

### EXAMPLES

### Example 1:

### 1.1.1 Influenza Antigen Used

Purified monovalent A/Beijing8-9/93 (H3N2) and A/Johannesburg/97 (H1N1) split virus influenza antigens provided by Chiron Vaccines, Sienna, Italy. Dosing was based on HA content as assayed by single radial immunodiffusion (SRID) as described previously (Johannsen R. et al. (1985) Vaccine 3:235-240). LT-K63 and LT-R72 were prepared as described previously (Pizza M. et al. (1994) Mol. Microbiol. 14:51-60). Wild-type LT (wtLT obtained from Sigma *(Escherichia coli* heat-labile enterotoxin, lyophilized powder, Sigma-Aldrich. St. Louis, MO. USA). All immunogen preparations were formulated in phosphate buffered saline (PBS). Immunogens prepared for i.g. admmistration included 1.5% w:v sodium bicarbonate.

### 1.2.1 Immunization and Sample Collection

Groups of 10 female Balb/c mice (Charles River Labs, Wilmington, MA), 6 to 10 weeks old, were immunized according to Tables 1, 2, and 3. Mice were fasted 12 hours prior to each immunization. Immunizations were made either by i.m. (50 µl) injection into the posterior thigh muscle or direct i.g. into the stomach (200 µl) using a 20-gauge stainless steel feeding needle attached to a 1 ml springe. Animals were not anesthetized during immunizations. Collection of blood samples were performed by sinus orbital puncture using a microhematocrit tube after light anesthesia using isofluorine gas. Serum was separated from blood using standard methods. Saliva wash (SW) samples were collected by placing one end of a 0.2 x 3.2 cm cellulose adsorbent wick (America Filtrona. Richmond, V.A) into the mouth of each unanesthetized mouse for one to two minutes to adsorb saliva. Antibodies were then eluted into PBS (400 µl) before assay. Nasal wash samples (NW) were collected by first anesthetizing animals with a mixture of ketamine hydrochloride (80 mg/kg) and xylazine (4 mg/kg). PBS (600 µl) was inserted into the nasal cavity using a catheter connected to a small syringe while the animal was held in a dorsal recumbent position with the head tilted slightly downward. Washes were collected by gravity flow into small tubes. The serum and secretory samples were stored frozen (-70°C) until assayed.

### 1.2.2 Effect of enterotoxin type and dose on antibody responses after i.g. immunization

A dose ranging study was conducted to determine the dose response relationship for LT-K63 and LT-R72 for i.g. immunization with A/Beijing8-9/93 HA. Groups of 10 mice were immunized by the i.g. route with 20 ug of A/Beijing8-9/93 HA in combination with three dose levels of wtLT, LT-K63, and LT-R72 as described in Table 1. Groups that received A/Beijing8-9/93 adjuvanted with wtLT, and a group that received unadjuvanted A/Beijing8-9/93 HA (HA only) were included for comparison purposes.

**TABLE 1. Dose ranging of adjuvants**

| Group | Amt (mg) of: | | | Immunization Schedule (days) | Route | Day of sample collection |
|---|---|---|---|---|---|---|
| | HA | Enterotoxin | Enterotoxin Type | | | |
| 1 | 20 | None | - | 0, 21, 35 | i.g. | 49 |
| 2 | 20 | 1 | wtLT | 0, 21, 35 | i.g. | 49 |
| 3 | 20 | 10 | wtLT | 0, 21, 35 | i.g. | 49 |
| 4 | 20 | 25 | wtLT | 0, 21, 35 | i.g. | 49 |
| 5 | 20 | 1 | LT-K63 | 0, 21, 35 | i.g. | 49 |
| 6 | 20 | 10 | LT-K63 | 0, 21, 35 | i.g. | 49 |
| 7 | 20 | 100 | LT-K63 | 0, 21, 35 | i.g. | 49 |
| 8 | 20 | 1 | LT-R72 | 0, 21, 35 | i.g. | 49 |
| 9 | 20 | 10 | LT-R72 | 0, 21, 35 | i.g. | 49 |
| 10 | 20 | 100 | LT-R72 | 0, 21, 35 | i.g. | 49 |

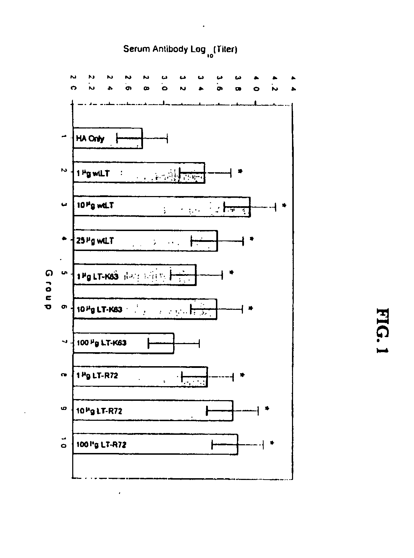

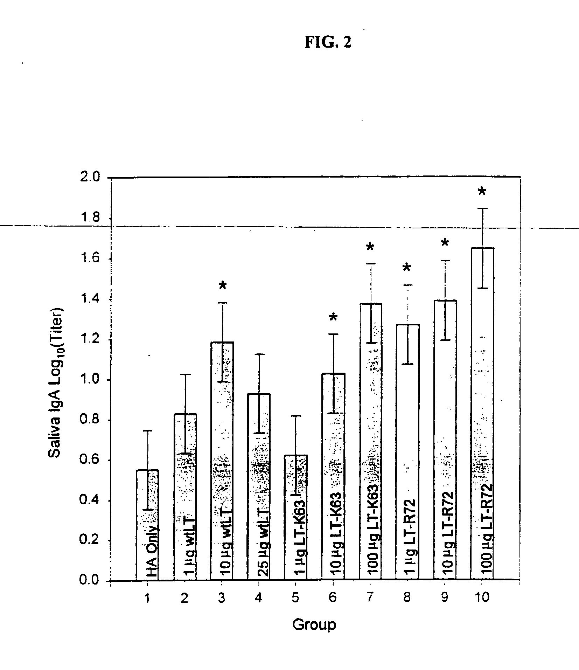

The results are shown in Figures 1 and 2.

### 1.2.3 Effect of A/Beijing8-9/93 HA dose on antibody responses at two dose levels of LT-R72

A second dose ranging study was conducted to determine the optimum dose of A/Beijing8-9/93 HA for i.g. immunization when adjuvanted with LT-R72. Groups of 10 mice were immunized by the i.g. route with three dose levels of A/Beijing8-9/93 HA in combination with either 10 µg or 100 µg LT-R72 as described in Table 2. An unadjuvanted A/Beijing8-9/93 HA control group (HA only) was included for comparison purposes.

**TABLE 2. Antigen dose ranging with LT-R72**

| Group | Amt (mg) of: | | | Immunization Schedule (days) | Route | Day of sample collection |
|---|---|---|---|---|---|---|
| | HA | Enterotoxin | Enterotoxin Type | | | |
| 11 | 20 | None | - | 0, 21, 35 | i.g. | 49 |
| 12 | 1 | 10 | LT-R72 | 0, 21, 35 | i.g. | 49 |
| 13 | 5 | 10 | LT-R72 | 0, 21, 35 | i.g. | 49 |
| 14 | 20 | 10 | LT-R72 | 0, 21, 35 | i.g. | 49 |
| 15 | 1 | 100 | LT-R72 | 0, 31, 35 | i.g. | 49 |
| 16 | 5 | 100 | LT-R72 | 0, 21, 35 | i.g. | 49 |
| 17 | 20 | 100 | LT-R72 | 0, 21, 35 | i.g. | 49 |

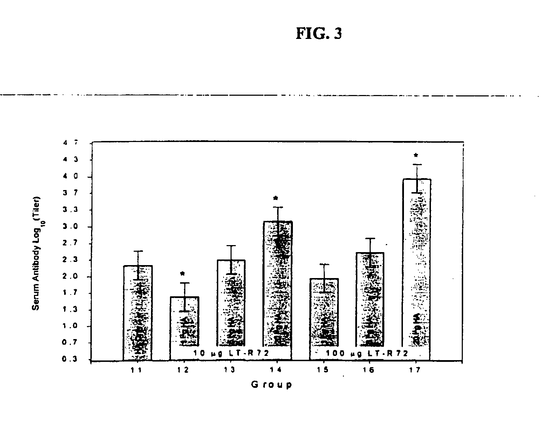

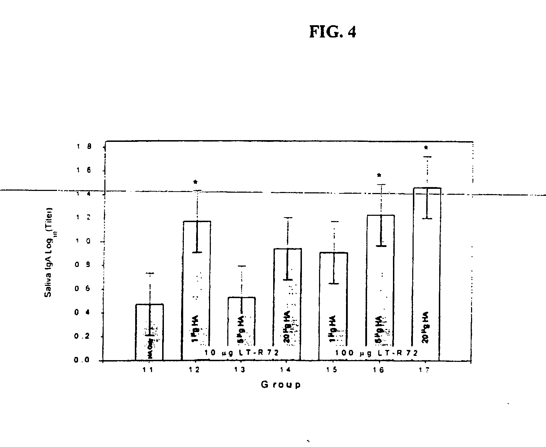

The results are shown in Figures 3 and 4.

### 1.1.4 Comparison of i.g. and i.m. immunization

The serum antibody responses of mice i.g. immunized with A/Johannesburg/97 HA either alone or in combination with an LT were compared to mice immunized with A/Johannesburg/97 HA by the i.m. route. Groups of 10 mice were immunized by the i.g. route with 20 µg A/Johannesburg/97 HA either alone, or in combination with two dose levels of wtLT. LT-K63. or LT-R72 as described in Table 3. A group receiving 1 µg A/Johannesburg/97 HA by the i.m. route was included for comparison purposes.

**TABLE 3. Comparison of intragastric verses intramuscular immunization**

| Group | Amt (mg) of: | | | Immunization Schedule (days) | Route | Day of sample collection |
|---|---|---|---|---|---|---|
| | HA | Enterotoxin | Enterotoxin Type | | | |
| 18 | 20 | None | - | 0, 21, 35 | i.g. | 49 |
| 19 | 20 | 1 | wtLT | 0, 21, 35 | i.g. | 49 |
| 20 | 20 | 10 | wtLT | 0, 21, 35 | i.g. | 49 |
| 21 | 20 | 10 | LT-K63 | 0, 21, 35 | i.g. | 49 |
| 22 | 20 | 100 | LT-K63 | 0, 21, 35 | i.g. | 49 |
| 23 | 20 | 10 | LT-R72 | 0, 21, 35 | i.g. | 49 |
| 24 | 20 | 100 | LT-R72 | 0, 21, 35 | i.g. | 49 |
| 25 | 1 | None | - | 0, 21, 35 | i.m. | 49 |

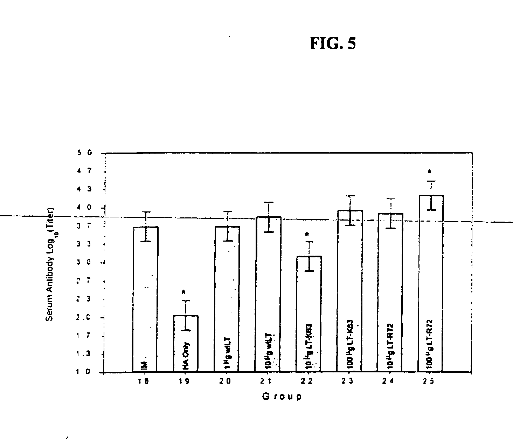

The results are shown in Figures 5, 6 and 7.

### 1.3.1 Antibody ELISA

Serum samples from individual animals were assayed for total anti-HA Ig (IgG plus IgA plus IgM) titers by a 3,3',5,5'-tetramethylbenzidine based colorimetric enzyme-linked immunosorbent assay (ELISA) as previously described with A/Bejing8-9/93 or A/Johannesburg/97 as appropriate as coating antigen Harlow E. and D. Lane "Immunoassay" in ANTIBODIES: A LABORATORY MANUAL. Cold Springs Harbor Laboratory, New York. 1988. pp. 553-612). A₄₉₀ was measured using a standard ELISA reader. The titers represent reciprocal serum dilutions giving an A₄₉₀ of 0.5 and were normalized to a serum standard assayed in parallel. SW and NW samples from individual animals were assayed for HA specific IgA titers using a bioluminescence immunosorbent assay as previously described with A/Bejing8-9/93 or A/Johannesburg/97 as appropriate as coating antigen (Ugozzoli M. *et al.* (1998) *Immunol.* 93:563-571). The goat anti-mouse IgA biotin conjugate (EY Labs, San Mateo, CA) used was pre-saturated with purified mouse IgG (1 mg/ml, Sigma Chemical Company, St. Louis, MO) to reduce cross-reactivity. Quantitation was based on the number of relative light units representing total luminescence integrated over 3 s (arbitrary units). Titers represent log dilution values linearly extrapolated from the log of the relative light units to a cutoff value at least two standard deviations above mean background.

### 1.3.2 Serum antibody responses after i.g. administration

Serum antibody responses (Fig. 1) were significantly higher in most cases in animals that received A/Beijing8-9/93 HA in combination with either of the enterotoxins tested compared to animals that received unadjuvanted A/Beijing8-9/93 HA. Figure 1 shows mean anti-A/Beijing8-9/93 HA antibody titers in the serum of mice immunized with 20 µg doses of A/Beijing8-9/93 HA antigen either alone (HA only) or in combination with enterotoxins as shown in Table 1. Asterisks indicate groups whose values are significantly greater than that of the HA only group (P ≤ 0.05). A dose response was not clearly demonstrated, although groups that received A/Beijing8-9/93 HA in combination with 10 µg and 100 µg LT-R72 were found to be comparable to the serum antibody responses of groups receiving A/Beijing8-9/93 HA in combination with wtLT. Only one group (group 7 at a dose level of 100 µg LT-K63) did not demonstrate a strong adjuvant effect.

A clearer adjuvant dose response was found in the antigen-specific saliva IgA responses (Fig. 2). Figure 2 shows mean anti-A/Beijing8-9/93 HA SW IgA antibody titers of groups of mice immunized with 20 µg doses of A/Beijing8-9/93 HA antigen either alone (HA only) or in combination with enterotoxins as shown in Table 1. Asterisks indicate groups whose values are significantly greater that that of the HA only group (P≤0.05). Significantly stronger saliva IgA responses were demonstrated for all but one of the LT-K63 and LT-R72 adjuvanted groups compared to animals that received A/Beijing8-9/93 HA alone. Additionally, animals dosed i.g. with 20 µg A/Beijing8-9/93 HA in combination with 100 µg LT-R72 were found to have a significantly higher (P ≤ 0.05) antigen specific saliva IgA response than animals dosed i.g. with either 10 µg or 25 µg wtLT.

### 1.3.3 Comparison of Enterotoxic dose on antigen-specific SW IgA responses after i.g. administration

The antigen-specific serum antibody responses (Fig. 3) demonstrated a dose response trend with respect to the dose level of A/Beijing8-9/93 and the dose level of LT-R72 the animals were immunized with. Figure 3 shows mean anti-A/Beijing8-9/93 HA antibody titers in the serum of mice immunized with 1, 5, or 20 µg doses of A/Beijing8-9/93 HA in combination with either 10 µg or 100 µg of LT-R72 as compared to 20 µg HA administered alone (HA only) as shown in Table 2. Asterisks indicate groups whose values are significantly different than that of the HA only group (P ≤ 0.05). Serum antibody responses were significantly higher (P ≤ 0.05) in animals immunized i.g. with 20 µg doses of A/Beijing8-9/93 HA in combination with either 10 µg or 100 µg LT-R72 as compared to the unadjuvanted control group. The group that received the highest dose level tested (20 µg A/Beijing8-9/93 in combination with 100 µg LT-R72) had a significantly higher (P ≤ 0.05) antigen-specific serum antibody response than all other groups tested.

The antigen-specific saliva IgA responses (Fig. 4) matched the trend seen with the serum antibody responses with the exception of the group that received 1 µg A/Beijing8-9/93 in combination with 10 µg LT-R72 (group 12). Figure 4 shows mean anti-A/Beijing8-9/93 HA SW IgA antibody titers of groups of mice immunized with 1, 5, or 20 µg doses of A/Beijing8-9/93 HA in combination with either 10 µg or 100 µg of LT-R72 as compared to 20 µg HA administered alone (HA only) as shown in Table 2. Asterisks indicate groups whose values are significantly greater than that of the HA only group (P ≤ 0.05). Animals that received either 5 µg or 20 µg A/Beijing8-9/93 HA in combination with 100 µg LT-R72 demonstrated a significantly higher (P ≤ 0.05) antigen-specific saliva IgA response compared to animals that received unadjuvanted A/Beijing8-9/93 HA.

### 1.3.4 Comparison of the effects of i.m. and i.g. administration on antigen-specific serum antibody responses

Serum antigen-specific antibody responses (Fig. 5) were equivalent or higher for mice immunized i.g. with 20 µg A/Johannesburg/97 HA adjuvanted with an LT compared to mice i.m. immunized. Figure 5 shows mean anti-A/Johannesburg/97 HA antibody titers in the serum of mice immunized as shown Table 3. Asterisks indicate groups whose values are significantly different than that of the i.m. immunized group (P ≤ 0.05). Mice i.g. immunized with either 20 µg A/Johannesburg/97 HA unadjuvanted (HA only) or in combination with 10 µg LT-K63 were significantly lower (P ≤ 0.05) in serum antigen specific antibody responses compared to mice immunized by the i.m. route, nevertheless, i.g. immunization in the presence of 10 µg LT-K63 resulted in a log higher antibody responses than i.g. immunization with unadjuvanted A/Johannesburg/97 HA. Mice i.g. immunized with 20 µg A/Johannesburg/97 HA in combination with 100 µg LT-R72 resulted in serum antigen-specific antibody responses that were significantly (P ≤ 0.05) higher than found for i.m. immunization.

### 1.3.5 Comparison of the effects of i.m. and i.g. administration on antigen-specific NW IgA antibody responses

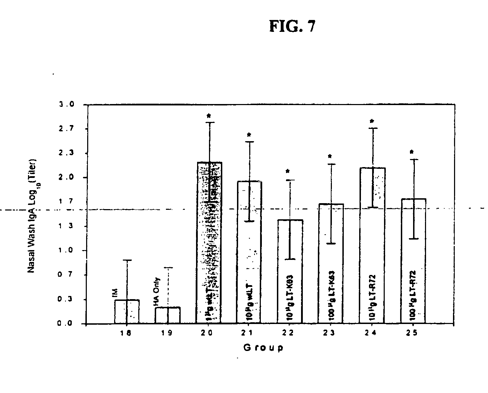

Figure 7 shows a comparison of the effects of i.m. and i.g. administration of A/Johannesburg/97 HA on antigen-specific NW IgA antibody responses. Shown are mean anti-A/Johannesburg/97 HA NW IgA antibody titers of mice immunized as shown Table 3. Asterisks indicate groups whose values are significantly greater than that of the i.m. immunized group (P ≤ 0.05). Antigen-specific NW IgA responses were found to be significant only in those mice immunized i.g. with 20 µg A/Johannesburg/97 HA in combination with either wtLT. LT-K63, or LT-R72. Mice immunized by i.m. immunization, or i.g. with 20 µg A/Johannesburg/97 HA alone did not result in significant antigen-specific NW IgA responses. No significant differences were seen in the antigen-specific IgA responses as a consequence of enterotoxin dose level or type.

### 1.4.1 HI Assay.

Serum samples pooled by group were assayed for hemagglutination inhibition (HI) titer by the Viral and Rickettsial Disease Laboratory (Department of Health Services, Berkeley, CA) using a standard ELISA. The HI assay is based on the ability of sample sera to inhibit the agglutination of goat erythrocytes in the presence of HA antigen. The resulting titers are expressed as the reciprocal dilution required for complete inhibition (Hierholzer J.C. and M.T. Suggs (1969) Appl. Microbiol. 18:816-823; Hierholzer J.C. et al. (1969) Appl. Microbiol. 18:824-33).

### 1.4.2 Comparison of the effects of i.m. and i.g. administration on serum HI titers

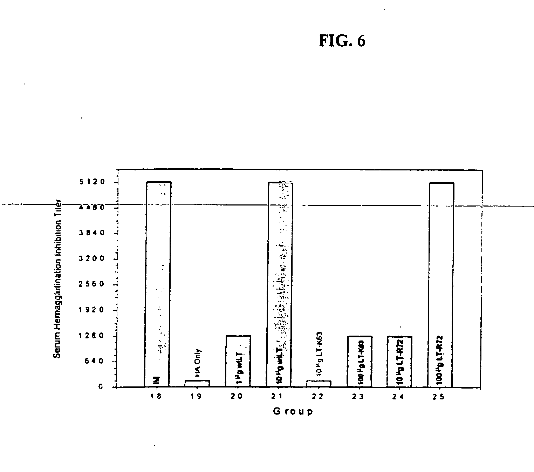

Figure 6 shows a comparison of the effects of i.m. and i.g. administration of A/Johannesburg/97 HA on serum HI titers. The data shown is for pooled serum from groups of mice immunized as shown Table 3. Serum HI titers for mice i.g. immunized with 20 µg A/Johannesburg/97 HA in combination with either 10 µg wtLT. or 100 µg LT-R72 were comparable in potency to mice i.m. immunized. Mice that were i.g. immunized with 20 µg A/Johannesburg/97 HA in combination with either 1 µg wtLT, 10 µg LT-R72, or 100 µg LT-K63 resulted in modest HI titer levels. Significant HI titers were not demonstrated for mice i.g. immunized with 20 µg A/Johannesburg/97 HA either alone, or in combination with 10 µg LT-K63.

### 1. 5. 1 Statistics.

Log anti-A/Beijing8-9/93 and anti-A/Johannesburg/97 HA serum Ig, saliva IgA, and nasal IgA titers from individual animals were analyzed using a Fisher least significant-difference procedure (Andrews H.P. et al. (1980) Am. Statistician 34:195-199). Comparison intervals were presented such that non-overlapping bars imply a statistical significance between means of greater than 5% (P ≤ 0.05).

The above experiements demonstrate that potent antigen-specific serum antibody and viral neutralizing titers (as indicated by HI titers) are comparable or are stronger than i.m. IgA responses when induced in mice with influenza HA antigens using i.g. immunization and adjuvanting with mutant LT's that demonstrate significantly reduced (LT-R72) and unmeasurable levels (LT-K63) of ADP-ribosyltransferase activity (Giuliani M.M. et al. (1998) J. Exp. Med. 187:1123-1132).

The foregoing examples are illustrative of the invention, but are not intended to be limiting of the scope of the invention which is limited by the appended claims.

## Claims

1. A composition comprising
(i) an immunogenic amount of a hemagglutinin antigen of an influenza virus and
(ii) at least one heat-labile, mutant *Escherichia coli* enterotoxin selected from the group consisting of LT-K63 and LT-R72,
**characterised in that** the composition is for oral administration to a mammal and is in the form of ingestible tablets, buccal tablets, troches, capsules, elixirs, syrups or wafers.

2. The composition of claim 1 wherein the LT-K63 or LT-R72 are present at a total dose of between 10µg and 10 mg.

3. The composition of claim 2, in the form of ingestible tablets or buccal tablets.

4. The composition of any preceding claim, wherein the mammal is a human.

5. Use of
(i) a hemagglutinin antigen of an influenza virus and
(ii) a heat-labile, mutant *Escherichia coli* enterotoxin selected from the group consisting of LT-K63 and LT-R72,
in the manufacture of a medicament for oral administration to a mammal to elicit an immune response.

6. Use according to claim 5 wherein administration to said mammal elicits antigen-specific IgA in nasal secretions or saliva of said mammal.

7. Use according to claim 5 or claim 6 wherein the LT-K63 or LT-R72 are present in the medicament at a total dose of 10µg to 10mg.

8. Use according to any one of claims 5 to 7 wherein the medicament is in the form of ingestible tablets, buccal tablets, troches, capsules, elixirs, suspensions, syrups or wafers.

9. Use according to claim 8 wherein the medicament is in the form of ingestible tablets or buccal tablets.

10. The use of any one of claims 5 to 9, wherein the mammal is a human.

11. The composition of any one of claims 1 to 4, for use in medicine.

## Patentansprüche

1. Zusammensetzung, umfassend
(i) eine immunogene Menge eines Hämagglutinin-Antigens eines Influenzavirus und
(ii) wenigstens ein hitzelabiles, mutiertes Escherichia coli-Enterotoxin, das aus der aus LT-K63 und LT-R72 bestehenden Gruppe ausgewählt ist,
**dadurch gekennzeichnet, dass** die Zusammensetzung für die orale Verabreichung an einen Säuger vorgesehen ist und in Form von einnehmbaren Tabletten, Bukkaltabletten, Pastillen, Kapseln, Elixieren, Sirupen oder Oblaten vorliegt.

2. Zusammensetzung nach Anspruch 1, wobei LT-K63 bzw. LT-R72 in einer Gesamtdosis zwischen 10 µg und 10 mg vorliegen.

3. Zusammensetzung nach Anspruch 2, in Form von einnehmbaren Tabletten oder Bukkaltabletten.

4. Zusammensetzung nach einem vorhergehenden Anspruch, wobei es sich bei dem Säuger um einen Menschen handelt.

5. Verwendung
(i) eines Hämagglutinin-Antigens eines Influenzavirus und
(ii) eines hitzelabilen, mutierten Escherichia coli-Enterotoxins, das aus der aus LT-K63 und LT-R72 bestehenden Gruppe ausgewählt ist,
bei der Herstellung eines Arzneimittels zur oralen Verabreichung an einen Säuger zum Hervorrufen einer Immunantwort.

6. Verwendung nach Anspruch 5, wobei die Verabreichung an den Säuger antigenspezifisches IgA in Nasensekreten oder Speichel des Säugers hervorruft.

7. Verwendung nach Anspruch 5 oder Anspruch 6, wobei LT-K63 bzw. LT-R72 im Arzneimittel in einer Gesamtdosis von 10 µg bis 10 mg vorliegen.

8. Verwendung nach einem der Ansprüche 5 bis 7, wobei das Arzneimittel in Form von einnehmbaren Tabletten, Bukkaltabletten, Pastillen, Kapseln, Elixieren, Suspensionen, Sirupen oder Oblaten vorliegt.

9. Verwendung nach Anspruch 8, wobei das Arzneimittel in Form von einnehmbaren Tabletten oder Bukkaltabletten vorliegt.

10. Verwendung nach einem der Ansprüche 5 bis 9, wobei es sich bei dem Säuger um einen Menschen handelt.

11. Zusammensetzung nach einem der Ansprüche 1 bis 4, zur Verwendung in der Medizin.

## Revendications

1. Composition comprenant
(i) une quantité immunogène d'un antigène d'hémagglutinine d'un influenzavirus, et
(ii) au moins une entérotoxine *d'Escherichia coli* mutante thermolabile choisie dans le groupe constitué par LT-K63 et LT-R72,
**caractérisée en ce que** la composition est destinée à une administration orale à un mammifère et se trouve sous la forme de comprimés ingérables, de comprimés buccaux, de trochisques, de capsules, d'élixirs, de sirops ou de cachets.

2. Composition selon la revendication 1, **caractérisée en ce que** la LT-K63 ou la LT-R72 est présente selon une dose totale comprise entre 10 µg et 10 mg.

3. Composition selon la revendication 2, sous la forme de comprimés ingérables ou de comprimés buccaux.

4. Composition selon l'une quelconque des revendications précédentes, **caractérisée en ce que** le mammifère est un être humain.

5. Utilisation
(i) d'un antigène d'hémagglutinine d'un influenzavirus, et
(ii) d'une entérotoxine *d'Escherichia coli* mutante thermolabile choisie dans le groupe constitué par LT-K63 et LT-R72,
dans l'élaboration d'un médicament destiné à une administration orale à un mammifère, afin de déclencher une réponse immunitaire.

6. Utilisation selon la revendication 5, **caractérisée en ce que** l'administration audit mammifère déclenche un IgA spécifique d'un antigène dans les sécrétions nasales ou la salive dudit mammifère.

7. Utilisation selon la revendication 5 ou la revendication 6, **caractérisée en ce que** la LT-K63 ou la LT-R72 est présente dans le médicament selon une dose totale allant de 10 µg à 10 mg.

8. Utilisation selon l'une quelconque des revendications 5 à 7, **caractérisée en ce que** le médicament se trouve sous la forme de comprimés ingérables, de comprimés buccaux, de trochisques, de capsules, d'élixirs, de suspensions, de sirops ou de cachets.

9. Utilisation selon la revendication 8, **caractérisée en ce que** le médicament se trouve sous la forme de comprimés ingérables ou de comprimés buccaux.

10. Utilisation selon l'une quelconque des revendications 5 à 9, **caractérisée en ce que** le mammifère est un être humain.

11. Composition selon l'une quelconque des revendications 1 à 4, pour une utilisation en médecine.
